# EUROPEAN PATENT SPECIFICATION

(11) **EP 0 759 171 B1**
(45) Date of publication and mention of the grant of the patent: **15.04.1998**
(21) Application number: 95921747.2
(22) Date of filing: 12.05.1995
(51) Int. Cl.: G01N 33/535, C12Q 1/42, G01N 27/327

(54) **PARACETAMOL PHOSPHATE FOR IMMUNOASSAYS**
PARACETAMOLPHOSPHAT FÜR IMMUNOASSAYS
PHOSPHATE DE PARACETAMOL POUR DOSAGES IMMUNOLOGIQUES

(30) Priority: 13.05.1994 GB 9409590
(43) Date of publication of application: 26.02.1997
(73) Proprietor: CAMBRIDGE LIFE SCIENCES PLC, Ely Cambridgeshire CB7 4DT (GB)
(72) Inventor: ATHEY, Dale, Newcastle-upon-Tyne NE3 2UG (GB); RAWSON, Keith, Cambridgeshire CB6 1ED (GB)
(74) Representative: Rupp, Herbert, Dr.
(86) International application number: EP9501814
(87) International publication number: WO9531724

(56) References cited:
- DE-A- 2 949 669
- CHEMICAL ABSTRACTS, vol. 119, no. 4, issued 1993, July 26, (Columbus, Ohio, USA), F. PARIENTE et al. "Alkaline phosphatase-modified carbon fiber disk microelectrode for the determination of 4-aminophenyl phosphate" page 873, no. 39 888w; & Anal.Chim.Acta 1993, 273(1-2), 187-93.
- CHEMICAL ABSTRACTS, vol. 122, no. 11, issued 1995, March 13 (Columbus, Ohio, USA), A.L.G.L. SALLE et al. "Determination of alkaline phosphatase using a Nafion*- -modified electrode", page 482, no. 127 077y; & J. Electroanal.Chem. 1994, 379(1-2), 281-91.
- CHEMICAL ABSTRACTS, vol. 122, no. 17, issued 1995, April 24, (Columbus, Ohio, USA), A.L. GHINDILIS et al. "Determination of p-amino- phenol and catecholamines at picomolar concentrations based on recycling enzyme amplification", page 533, no. 209 054c; & Anal.Chim.Acta 1995, 304(1), 25-31.

## Description

### Technical Field

This invention relates to a novel method for electrochemical detection of phosphatase activity.

### Background of the Invention

The excellent detection limits afforded by electrochemical techniques makes electrochemistry an attractive methodology to combine with the selectivity of an immunoassay. In enzyme linked immunosorbent assay (ELISA), enzymes such as alkaline phosphatase and horse radish peroxidase are coupled to either the analyte or to antibodies in two site assays, so that the latter can be determined from the activity of the enzyme. The detection of the enzyme activity in enzyme immunoassays is usually performed by spectroscopic measurements of chromogenic or fluorogenic substrates. However, the wide dynamic range, sensitivity and low cost of electrochemical apparatus represent an alternative attraction.

A number of rapid, simple enzyme electrodes have been developed for measurement of substances of clinical importance. Alkaline phosphatase is an important enzyme in both clinical chemistry and analytical chemistry. In addition, many biochemical determinations rely on alkaline phosphatase for detection and amplification. Both heterogeneous and homogeneous immunoassays using alkaline phosphatase have been described and antibody or antigen conjugates with alkaline phosphatase have become common in bioanalytical chemistry. Alkaline phosphatase has advantages over many other enzyme labels in both terms of its high turnover and also its broad specificity range. Until recently, alkaline phosphatase based immunoassay procedures have been concerned primarily with colorimetric substrates and in particular, 4-nitrophenyl phosphate.

The use of electrochemical detection for a number of alkaline phosphatase substrates has been reported, although most early work concerns the use of phenyl phosphate [Heineman et al., Anal. Chem., 57 (1985) 1321A]. Enzymatic hydrolysis of this substrate produces phenol which can be readily oxidised at potentials in excess of +800 mV vs Ag/AgCl. The system can be used in an assay involving a separation and a wash step. This method is generally not applicable to systems incorporating components which oxidise at this high potential.

4-Aminophenyl phosphate and 1-naphthyl phosphate have also been used as electrochemical substrates for alkaline phosphatase as the redox potential of the product of the enzymatic hydrolysis, 4-aminophenol and 1-naphthol, are lower than for phenol. However, the hydrolysis product of 4-aminophenyl phosphate, 4-aminophenol, is extremely unstable, oxidising and polymerising very rapidly at room temperature, leaving polymeric deposits on the electrode. This compound could not be used for prolonged incubation of alkaline phosphatase as a substrate. Whilst 1-naphthyl phosphate is relatively stable, the product 1-naphthol polymerises on the electrode surface when oxidised electrochemically making this an unsuitable substrate where the electrode is used for repeated measurements.

### Summary of the Invention

The object of this invention is to provide novel substrates for an electrochemical immunoassay which generate in the course of a sandwich or competitive or other immunoassay type by the use of any suitable enzyme an electrochemically active species which are readily oxidised at the electrode surface.

In the present invention, a novel method for the detection of phosphatase enzyme activity is described. It was found that using 4-acetamidophenyl phosphate (paracetamol phosphate) and N-hydroxy-4-acetamidophenyl phosphate gave rise to surprising advantages. The substrates 4-acetamidophenyl phosphate and N-hydroxy-4-acetamidophenyl phosphate are easy to prepare, offer good stability both as a powder and in solution, and their hydrolysis products, 4-acetamidophenol and N-hydroxy-4-acetamidophenol, respectively, do not impair the surface activity of the measuring electrode.

One aspect of the invention is the use of 4-acetamidophenyl phosphate and N-hydroxy-4-acetamidophenyl phosphate as substrates for electrochemical detection of phosphatase activity. A further aspect of the invention is a method of electrochemical detection of phosphatase activity characterised in that 4-acetamidophenyl phosphate or N-hydroxy-4-acetamidophenyl phosphate is used as substrate. The use of 4-acetamidophenyl phosphate as substrate is a preferred aspect of the invention. The detection of alkaline phosphatase is another preferred aspect of the invention.

Further aspects of the invention are evident from the claims.

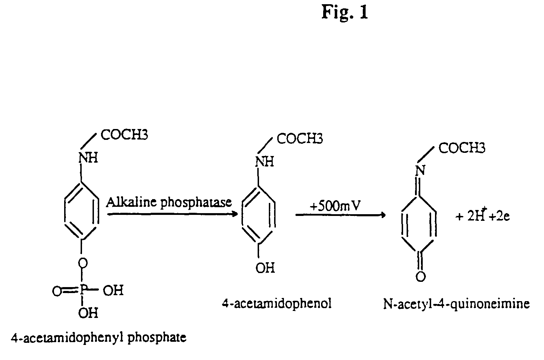

The 4-acetamidophenol and N-hydroxy-4-acetamidophenol are oxidised at the electrode surface producing 2 electrons. The reaction scheme of Figure 1 describes the chemical process for the oxidation of 4-acetamidophenol.

The electrochemical substrates according to this invention can be used in many different formats known to the man skilled in the art. The amount of product, 4-acetamidophenol and N-hydroxy-4-acetamidophenol, respectiveley, produced from the hydrolysis by alkaline phosphatase is proportional to the amount of bound label and is quantified by amperometry at suitably polarised electrodes such as porous graphite or planar carbon.

### Description of Figures

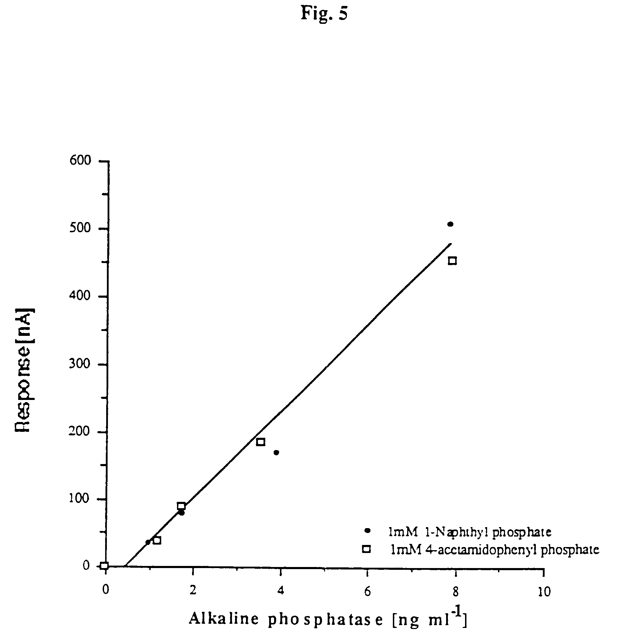

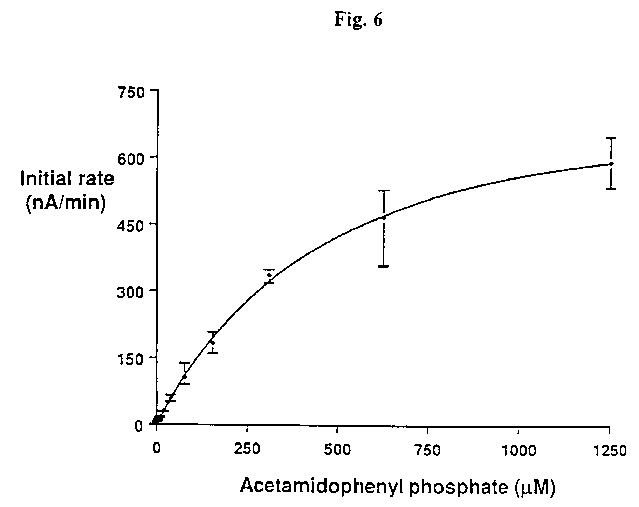

The invention is illustrated by figures 1 to 7:
- **Figure 1**: is showing the reaction scheme for 4-acetamidophenyl phosphate with alkaline phosphatase.
- **Figure 2**: is showing linear sweep voltammograms for 4-acetamidophenol and 1-naphthol.
- **Figure 3**: is showing the cyclic voltammogram of 4-acetamidophenyl phosphate before and after exposure to alkaline phosphatase.
- **Figure 4**: is showing an electrochemical calibration curve for 4-acetamidophenol and 1-naphthol.
- **Figure 5**: is showing the limit of alkaline phosphatase detection.
- **Figure 6**: is showing a Michaelis-Menten kinetic plot for the substrate 4-acetamidophenyl phosphate.
- **Figure 7**: is showing an electrochemical total T4 immunoassay standard curve.

### Examples

### Example 1

### Preparation of 4-acetamidophenyl phosphate

To 150 ml of acetic anhydride 11.0 g of disodium 4-aminophenyl phosphate are added. After stirring for 30 minutes, 400 ml of ether are added and the stirring is continued for a further 30 minutes. The precipitate is collected by filtration under reduced pressure and suspended in a mixture of 180 ml of acetone and 7 ml of water. After stirring for 15 minutes the precipitate is collected by filtration under reduced pressure and washed with acetone and ethanol. The residue is dissolved in 100 ml of hot water. The resulting solution is filtrated after addition of activated charcoal. With cooling some red substance precipitates. The clear solution is decanted from this precipitate and concentrated largely and stirred for 30 minutes under cooling with ice. The precipitate is collected by filtration under reduced pressure and treated with 50 ml of 1N hydrochloric acid at room temperature for one hour under stirring. The reaction mixture is then concentrated to about half the volume and stirred under cooling in the ice bath for 30 minutes. The resulting monohydrate of the title compound is collected by filtration and dried under reduced pressure (yield 6.50 g [55%]. m.p. 229°C with decomposition).

### Example 2

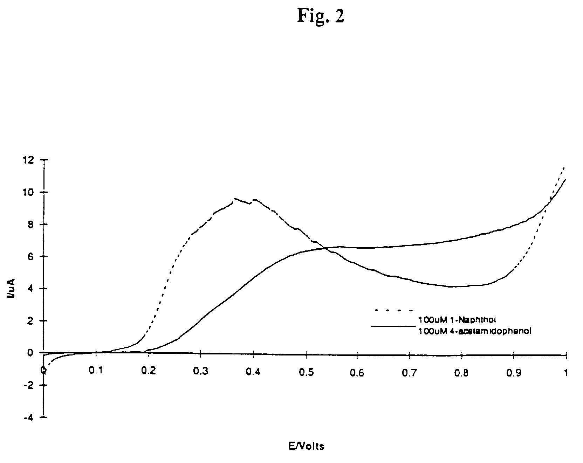

Linear sweep voltammetry was used to examine the electrochemistry of the 4-acetamidophenyl phosphate and its hydrolysis product. A flow-through coulometric cell was used with a disc of porous graphite, diameter 5 mm, thickness 1.7 mm (Toray Industries, Japan) as the working electrode. The contact diameter of the electrode with the liquid was 3 mm. Samples of 1-naphthol, 4-acetamidophenol and 4-acetamidophenyl phosphate in Tris buffer pH 9.6 were flowed through the cell at 300 µl/min. The potential was swept from 0 - 1 V at a rate of 1 mV/s, vs Ag/AgCl. The oxidation potential for 4-acetamidophenol was +500 mV and for 1-naphthol, +300 mV, which also showed a peak at this sweep rate due to fouling of the electrode surface (Figure 2).

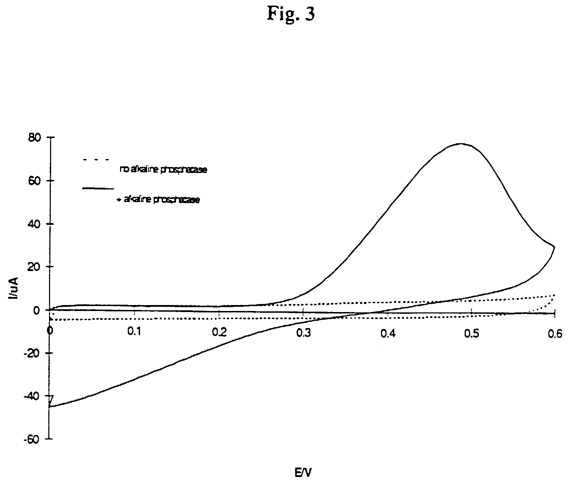

The second cyclic voltammogram of 4-acetamidophenyl phosphate substrate solution according to Figure 3 shows significant changes in electrochemistry after exposure to 200 µl of 2 µg/ml alkaline phosphatase (Biozyme, Newport). The oxidation peak after exposure to alkaline phosphatase was found to correspond to the 4-acetamidophenol oxidation peak.

### Example 3

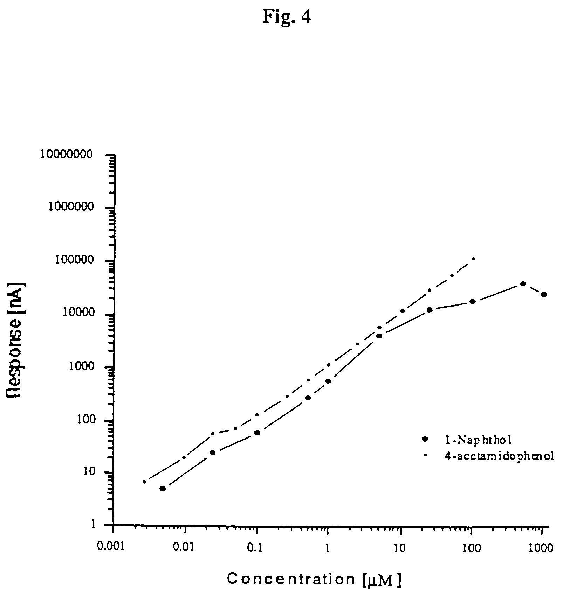

Calibration curves for 1-naphthol and 4-acetamidophenol were prepared in order to show the dynamic range of the alkaline phosphatase hydrolysis products. A flow-through coulometric cell was used with a disc of porous graphite, diameter 5 mm, thickness 1.7 mm (Toray Industries, Japan) as the working electrode. The contact diameter of the electrode with the liquid was 3 mm. Samples of 1-naphthol and 4-acetamidophenol in Tris buffer pH 9.6 were flowed through the cell at 300 µl/min with the electrode poised at +500 mV vs the Ag/AgCl reference electrode for 4-acetamidophenol and +300 mV for 1-naphthol. The resulting peak currents for each 200 µl sample were recorded and the peak area for 4-acetamidophenol. Figure 4 shows typical calibration curves for 1-naphthol and 4-acetamidophenol. Total oxidation of 4-acetamidophenol was proven from incorporating the peak area data in to Faraday's law.

### Example 4

In order to assess the limits of sensitivity that might be achieved using this approach, the hydrolysis of 4-acetamidophenyl phosphate by alkaline phosphatase was investigated.

Various dilutions of alkaline phosphatase were prepared in 50 mM diethanolamine buffer (pH 9.6) containing 1 mmol/l MgCl₂. These were then incubated for 5 minutes with 1 mmol/l solutions of 4-acetamidophenyl phosphate or 1-naphthyl phosphate before measuring the oxidation current using the flow-through cell.

The lower limit of detection was defined as the concentration of alkaline phosphatase corresponding to the mean plus 2 standard deviations of the blank response.

The lower limit of detection for alkaline phosphatase with 4-acetamidophenyl phosphate or 1-naphthyl phosphate as substrate is 0.9 ng/ml (7 x 10⁻¹⁰ mol/l).

### Example 5

The enzyme kinetic constants, Km and Vmax, were determined for three alkaline phosphatase substrates, 4-acetamidophenyl phosphate, 1-naphthyl phosphate and phenyl phosphate. A 10 mmol/l stock solution of each substrate was prepared in 10 ml of 50 mmol/l Tris buffer pH 10.

A stock solution of alkaline phosphatase was prepared by diluting the enzyme 1:1000 in buffer, to give a solution containing approximately 28 U of enzyme/ml. To perform the experiments the solution was diluted 1:10 to give a working enzyme solution at approximately 2.8 U/ml. The cell was set up as follows: The electrode was placed over the surface of the platinum button on the rank cell (hydroxyethyl cellulose was placed between platinum and carbon to improve conductivity and hold electrode in place) and a 0.05 µm polycarbonate membrane placed over the surface. The working electrode was polarised at +500 mV, +350 mV and +650mV vs Ag/AgCl for the substrates, 4-acetamidophenyl phosphate, 1-naphthyl phosphate and phenyl phosphate. To perform the kinetic experiments a background was obtained using 200 µl of the substrate solution and the reaction initiated using 20 µl of alkaline phosphatase solution. Initial rates were measured and as soon as the rate was obtained the cell washed out thoroughly with buffer.

| **Substrate** | Km (µM) | Vmax (nA/min) | Apparent kinetic constant Vmax/Km |
|---|---|---|---|
| 4-acetamidophenyl Phosphate | 651 | 1096 | 1.68 |
| 1-Naphthyl Phosphate | 725 | 981 | 1.35 |
| Phenyl Phosphate | 1307 | 1206 | 0.92 |

In comparison to the state of the art the above results indicate a favourable kinetic characteristics for the reaction between alkaline phosphatase and acetamidophenyl phosphate under the conditions described, and suggests the excellent applicability of this substrate to the immunoassay formats described.

### Example 6

A competitive immunoassay was prepared for total serum thyroxine (T4) using 4-acetamidophenyl phosphate as the substrate for the enzyme label. A T4-alkaline phosphatase (T4-ALP) conjugate was prepared from L-thyroxine sodium salt and linked to alkaline phosphatase (Biozyme) using disuccinimidyl suberate. The anti-T4 antibody was loaded at 2 µg/ml in carbonate buffer pH 9.6 on to 0.45 µm nitrocellulose membrane (Schleicher and Schuell) using an ELIFA membrane assay system (Pierce). The membrane was blocked and washed with Tris buffered saline pH 8.6.

Sample: conjugate (10:2) was run through the membrane in 2 minutes, and washed before exposure to substrate solutions.

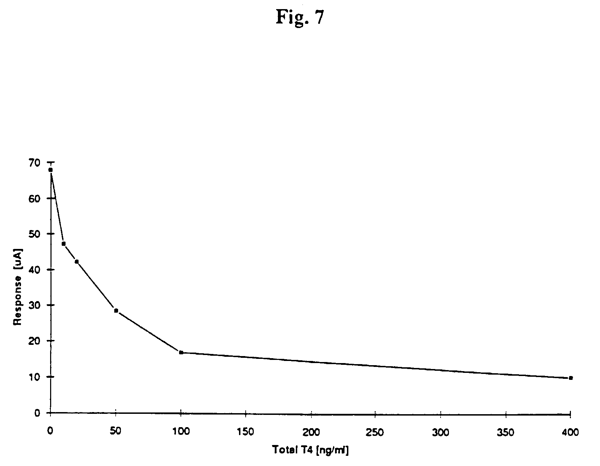

An electrochemical assay using 1 mmol/l 4-acetamidophenyl phosphate as substrate was performed by passing 200 µl through the membrane and flow-through cell. The product of phosphatase hydrolysis, 4-acetamidophenol, was detected at +500 mV vs Ag/AgCl reference electrode. Total assay time from sample addition was less than 10 minutes at room temperature (Figure 7).

## Claims

1. The use of 4-acetamidophenyl phosphate and N-hydroxy-4-acetamidophenyl phosphate as substrates for electrochemical detection of phosphatase activity.

2. The use of 4-acetamidophenyl phosphate and N-hydroxy-4-acetamidophenyl phosphate as substrates for electrochemical detection of alkaline phosphatase activity.

3. Method of electrochemical detection of phosphatase activity characterised in that 4-acetamidophenyl phosphate and N-hydroxy-4-acetamidophenyl phosphate are used as substrates.

4. Method according to claim 3, characterised in that 4-acetamidophenol and N-hydroxy-4-acetamidophenol, produced from 4-acetamidophenyl phosphate and N-hydroxy-4-acetamidophenyl phosphate, respectively, which are enzymatically hydrolysed by a phosphatase, are quantified by amperometry at suitably polarised electrodes.

5. Method according to claim 4, characterised in that the phosphatase is alkaline phosphatase.

## Patentansprüche

1. Verwendung von 4-Acetamidophenylphosphat und N-Hydroxy-4-acetamidophenylphosphat als Substrate für den elektrochemischen Nachweis von Phosphataseaktivität.

2. Verwendung von 4-Acetamidophenylphosphat und N-Hydroxy-4-acetamidophenylphosphat als Substrate für den elektrochemischen Nachweis der Aktivität von alkalischer Phosphatase.

3. Verfahren zum elektrochemischen Nachweis von Phosphataseaktivität, dadurch gekennzeichnet, daß man als Substrate 4-Acetamidophenylphosphat und N-Hydroxy-4-acetamidophenylphosphat einsetzt.

4. Verfahren nach Anspruch 3, dadurch gekennzeichnet, daß man aus 4-Acetamidophenylphosphat bzw. N-Hydroxy-4-acetamidophenylphosphat durch enzymatische Hydrolyse mit einer Phosphatase hergestelltes 4-Acetamidophenol und N-Hydroxy-4-acetamidophenol amperometrisch an geeignet polarisierten Elektroden quantitativ nachweist.

5. Verfahren nach Anspruch 4, dadurch gekennzeichnet, daß man alkalische Phosphatase einsetzt.

## Revendications

1. Utilisation du phosphate de 4-acétamidophényle et du phosphate de N-hydroxy-4-acétamidophényle en tant que substrats pour une détection électrochimique de l'activité de la phosphatase.

2. Utilisation du phosphate de 4-acétamidophényle et du phosphate de N-hydroxy-4-acétamidophényle en tant que substrats pour une détection électrochimique de l'activité de la phosphatase alcaline.

3. Procédé de détection électrochimique de l'activité de la phosphatase, caractérisé en ce que l'on utilise le phosphate de 4-acétamidophényle et le phosphate de N-hydroxy-4-acétamidophényle comme substrats.

4. Procédé selon la revendication 3, caractérisé en ce que l'on quantifie par ampérométrie à des électrodes polarisées d'une manière appropriée, le 4-acétamidophénol et le N-hydroxy-4-acétamidophénol produits respectivement à partir du phosphate de 4-acétamidophényle et du phosphate de N-hydroxy-4-acétamidophényle par une hydrolyse enzymatique par une phosphatase.

5. Procédé selon la revendication 4, caractérisé en ce la phosphatase est la phosphatase alcaline.
